# EUROPÄISCHE PATENTSCHRIFT

(11) **EP 1 280 482 B1**
(45) Veröffentlichungstag und Bekanntmachung des Hinweises auf die Patenterteilung: **07.11.2007**
(21) Anmeldenummer: 00987187.2
(22) Anmeldetag: 24.11.2000
(51) Int. Cl.: A61F 2/64, A61F 2/68, F16H 21/12

(54) **MEHRACHSIGES KÜNSTLICHES KNIEGELENK**
MULTIAXIS ARTIFICIAL KNEE JOINT
ARTICULATION ARTIFICIELLE MULTIAXIALE DU GENOU

(30) Priorität: 21.12.1999 DE 19961915; 29.05.2000 DE 10026440
(43) Veröffentlichungstag der Anmeldung: 05.02.2003
(73) Patentinhaber: OHIO WILLOW WOOD COMPANY, Mount Sterling, OH 43143 (US)
(72) Erfinder: LÜHRS, Bernd, 33142 Büren (DE); THEILE, Frank, 34519 Diemelsee Adorf (DE); GRIESSER, Michael, 33154 Salzkotten (DE); RICHARD, Hans, Albert, 33100 Paderborn (DE); KULLMER, Gunter, 33184 Altenbeken-Schwaney (DE)
(74) Vertreter: Holland, Ralf
(86) Internationale Anmeldenummer: PCT/DE2000/004180
(87) Internationale Veröffentlichungsnummer: WO 2001/045596

(56) Entgegenhaltungen:
- EP-A- 0 590 386
- EP-A- 0 947 182
- DE-C- 841 190
- FR-A- 1 187 444
- GB-A- 1 533 796
- US-A- 3 823 424
- US-A- 5 314 498
- US-A- 5 728 173
- US-A- 5 904 721

## Beschreibung

Die Erfindung betrifft ein mehrachsiges Gelenk, insbesondere ein künstliches Kniegelenk, dessen Drehachsen senkrecht auf einer Ebene stehen.

In der Prothetik sind um eine einzige Achse verschwenkende künstliche Kniegelenke bekannt, deren jeweilige Schwenkbewegung unbefriedigend verläuft und die insbesondere einen unnatürlichen Gehverlauf nur zulassen. Es sind deshalb mehrachsige Kniegelenke bekannt geworden, deren Achsen auf einer Ebene senkrecht stehen, beispielsweise gemäß US 5,314,498 und FR 1 187 444.

Bei derartigen mehrachsigen Kniegelenken beschreibt eine Polkurve während des Verschwenkens die Relativbewegung des Momentandrehpunktes. Hierdurch wird ein harmonisches Gangbild erzeugt, da das Begehen von unebenen Böden, das Bewältigen von kleineren Hindernissen und dergleichen durch eine Verkürzung des Kniegelenkes erleichtert wird. Hierzu ist bewußt das Bein anzuheben und erfolgt dann regelmäßig eine momentane Drehpunktverlagerung nach vorn, bevor das Gelenk in den eigentlichen Beugewinkel übergeht. Bei dieser nach vorn gerichteten Bewegung entfernen sich die Anlenkpunkte von einander, was eine Verlängerung des Kniegelenkes zur Folge hat. Hierfür ursächlich ist unter anderem, daß die Polkurve und damit der momentane Drehpunkt regelmäßig außerhalb des eigentlichen Kniegelenkes liegt. Insofern ist auch hier ein unnatürlicher Bewegungsablauf gegeben, da das an sich zu verkürzende Bein zunächst verlängert wird und deshalb höher zu heben ist, als es ein Hindernis oder der normale Bewegungsablauf es eigentlich erfordert.

Vor diesem technischen Hintergrund macht die Erfindung es sich zur Aufgabe, ein mehrachsiges Gelenk, insbesondere ein künstliches Kniegelenk, zur Verfügung zu stellen, welches einen harmonischen, der natürlichen Funktion eines Knies nachempfundenen Gang ermöglicht.

Zur Lösung dieser Aufgabe wird bei einem mehrachsigen Gelenk, insbesondere einem künstlichen Kniegelenk, dessen Drehachsen senkrecht auf einer Ebene stehen, darauf abgestellt, daß eine relative Verschwenkung einer oberen Plattform gegen eine untere Plattform aus einer Ruheposition in eine Position maximaler Auslenkung zugelassen wird, bei welcher Verschwenkung der Abstand der Plattformen sofort mit einer Auslenkung aus der Ruheposition verkürzt wird. In Folge wird die Polkurve, die die Relativbewegung des Momentandrehpunktes beschreibt, sofort auch eine Annäherung der beiden Plattformen, die jeweils an einen Ober- bzw. Unterschenkel angeschlossen sind, beschreiben. Damit wird bei einer Gehbewegung und einem Anheben der Prothese sofort der Abstand zwischen einem Boden und einem Fuß der Prothese soweit vergrößert, daß in nahezu natürlicher Weise Unebenheiten und kleiner Hindernisse bewältigt werden können.

Insbesondere ist hierbei auch daran gedacht, daß während des Verschwenkens der Plattformen gegeneinander der momentane Drehpunkt ständig innerhalb des eigentlichen Gelenkes liegt.

Hierbei wird unter den Plattformen jeweils ein Anschlußelement verstanden, das der Anbindung des erfindungsgemäßen Gelenks an einen Oberschenkelstumpf bzw. eine Unterschenkelprothese dient. Insoweit ist die räumliche Gestaltung der Plattformen offen.

In konstruktiver Ausgestaltung kann vorgesehen sein, daß das Gelenk in der Ruheposition und/oder der Position der maximalen Auslenkung selbstsichernd oder selbsthemmend ausgelegt ist oder diese Positionen durch Totpunktstellungen des Gelenkes markiert sind. Hieraus ergibt sich der Vorteil insbesondere in der Ruhestellung, daß das Gelenk auch bei Belastungen in der jeweiligen Stellung gleichsam verriegelt ist. Hieraus resultiert zum einen ein sicherer und entspannter Stand und wird zum anderen ein Einknicken des Gelenkes bei Fersenkontakt im Schrittzyklus vermieden. Hierbei ist insbesondere an eine Hemmung des Gelenks in einem Bereich einer Auslenkung aus einer der Positionen zwischen 0° und 10° gedacht. Jedoch ist das Einleiten der Beugephase aus der Ruheposition heraus, insbesondere auch bei 0° beginnend, problemlos möglich.

Alternativ oder zusätzlich kann vorgesehen sein, daß die Ruheposition und/oder die Position der maximalen Auslenkung durch einen oder mehrere Anschläge festgelegt ist bzw. sind. Solche Anschläge stellen unbedingt sicher, daß das Verschwenken lediglich in einem vorgegebenen Winkelbereich stattfinden kann. Auch kann durch einen Anschlag eine erheblich größere Belastung von dem Gelenk in den genannten Positionen aufgenommen werden.

Als zweckmäßig hat es sich erwiesen, wenn ein gedämpftes Einlaufen in die Ruheposition und/oder die Position der maximalen Auslenkung vorgesehen ist. Hierbei kann zum einen daran gedacht sein, spezielle Dämpfer beispielsweise auch an Anschlägen vorzusehen oder anderweitig die Winkelgeschwindigkeit der Drehachsen beim Einlaufen in die Positionen zu reduzieren. Auch kann durch die vorgegebene Kinematik des Gelenkes selbst, beispielsweise durch das Einlaufen in selbstsichernde, selbsthemmende Bereiche oder Totpunktlagen, ein gedämpftes, weiches Einlaufen in eine der Positionen erreicht werden.

In konstruktiver Ausgestaltung ist vorgesehen, daß die Plattformen durch zwei jeweils endseitig an den Plattformen angelenkte Hebel unterschiedlicher Länge verbunden sind.

Diese Maßnahme stellt sicher, daß die obere Plattform nicht nur in der Höhe variierend gegenüber der unteren verschoben wird, sondern es werden durch diese Maßnahme die Plattformen tatsächlichen um einen Winkel gegeneinander verschwenkt.

Als weitere konstruktive Maßnahme kann vorgesehen sein, daß in einer Position, insbesondere in der Ruheposition, der Öffnungswinkel der die Achsen der Hebel verbindenden Linien weniger als 95° beträgt, insbesondere können diese auch annähernd aufeinander senkrecht stehen. Bevorzugt ist hierbei daran gedacht, daß insbesondere in dieser Position auf der Verbindungslinie der Achsen eines ersten Hebels eine Achse des zweiten Hebels im wesentlichen liegend angeordnet ist. Hierdurch wird eine Stellung des Gelenkes erreicht derart, daß bei einem normalen Stehen, wenn das Gelenk die Ruheposition einnimmt, ein sicherer und entspannter Stand gewährleistet ist. Dies bis hin zu einer bestimmten Auslenkung, wenn dann eine Belastung in dieser Auslenkung erfolgt.

Hierzu ist weiter bevorzugt vorgesehen, daß während des Verschwenkens sich die Hebel überkreuzen oder zumindest in Verlängerung die Verbindungslinien der ersten und zweiten Achsen. Durch diese Maßnahmen wird unter anderem erreicht, daß der momentane Drehpunkt während des Verschwenkens innerhalb des Gelenkes nach der Erfindung liegt und sich infolge ein annähernd natürlicher Bewegungsablauf ergibt.

In weiterer Ausgestaltung ist vorgesehen, daß in einer Position, insbesondere in der Ruheposition, die an der unteren Plattform angelenkten ersten Achsen der Hebel im wesentlichen auf gleicher Höhe liegend angeordnet sind oder alternativ, daß eine an der unteren Plattform angelenkte erste Achse eines Hebels höher liegend angeordnet ist als die erste Achse des anderen Hebels. Gegenüber der von diesen beiden ersten Achsen aufgespannten Ebene ist bevorzugt in einer Position, insbesondere in der Ruheposition, zwischen dem ersten Hebel und dieser Ebene ein Winkel zwischen 45° und 75°, insbesondere zwischen 50° und 60° aufgespannt. Es ist dies der Winkel zwischen der die Achsen des ersten Hebels verbinden Linie und der die ersten Achsen verbinden Linie in der unteren Plattform.

Diese konstruktiven Maßnahmen orientieren sich an der natürlichen Anbindung der Kreuzbänder am Unterschenkel sowie die natürliche Anlenkung des vorderen Kreuzbandes am Unterschenkel im Stand. Entsprechend dem Abstand der Anbindung des vorderen bzw. hinteren Kreuzbandes am Unterschenkel ist bevorzugt weiter vorgesehen, daß der Abstand der ersten Achsen zwischen 36 mm und 62 mm beträgt. Ebenso kann der Abstand der ersten Achse zur zweiten, an der oberen Plattform angelenkten Achse des ersten Hebels, zwischen 45 mm und 80 mm betragen, womit wiederum die Länge eines vorderen Kreuzbandes zwischen Ober- und Unterschenkel einer durchschnittlich großen Person aufgegriffen wird. Entsprechend des Abstandes der Anbindung der Kreuzbänder am Oberschenkel ist ein Abstand der zweiten Achsen zwischen 16 mm und 46 mm vorgesehen. Die Nachbildung des menschlichen Knies hinsichtlich der Kreuzbänder wird durch einen Abstand, entsprechend der Länge des hinteren Kreuzbandes, der ersten Achse zur zweiten, an der oberen Plattform angelenkten Achse des zweiten Hebels, zwischen 27 mm und 62 mm betragend komplettiert.

Infolge dieser konstruktiver Maßnahmen wird auch erreicht, daß in der Ruheposition, d. h. im Stand, die Linie der hauptsächlichen vertikalen Belastung des Gelenkes bevorzugt zwischen den beiden oberen Achsen liegend verläuft, ggfls. auch geringfügig, in Schwenkrichtung gesehen, vor der zweiten Achse des zweiten Hebels, welche Linie jedoch von den Hebeln tatsächlich gekreuzt wird.

Entsprechend der natürlichen Bewegung ist auch eine Begrenzung der Auslenkung des Gelenkes durch entsprechende Totpunktlagen oder Anschläge zwischen 130° und 175° vorgesehen.

Um eine ausreichende Stabilität des Kniegelenks nach der Erfindung sicherzustellen, insbesondere in der Ruheposition, kann als eine weitere konstruktive Maßnahme vorgesehen sein, einen Hebel als Doppelhebel auszuführen. Es kann dies der erste oder der zweite Hebel sein, gegebenenfalls können beide Hebel als Doppelhebel endseitig einer bspw. gemeinsamen Welle ausgebildet sein. Ein solches Gelenk genügt dann höchsten Belastungserfordernissen.

In einer bevorzugten Ausführungsform ist das Gelenk nach der Erfindung mit einem Vorholer versehen, der bei einer Entlastung des Gelenkes, beispielsweise bei einem Anheben des Beines, eine natürlich wirkende Schwenkbewegung einleitet. Ein solcher Vorholer ist zwischen oberer und unterer Plattform regelmäßig angeordnet, so daß auftretende Kräfte dort auch nur angreifen.

Es ist zweckmäßig, den Vorholer in der Ruheposition vertikal zwischen den Achsen der oberen Plattform anzubinden. Zum einen steht dort ausreichen Platz für konstruktive Maßnahmen zur Verfügung und ist weiter dort eine Krafteinleitung für ein Auslenken des Gelenkes aufgrund der Hebelanodnungen günstig.

In konstruktiver Ausgestaltung ist vorgesehen, daß wenigstens ein Hebel des Vorholers an einem der oberen Plattform vorstehenden Ansatz angelenkt ist. Infolge dieser Maßnahme ist auch bei einem Verschwenken des Gelenkes der Hebel frei von der oberen Plattform. Gegebenenfalls sind in dieser noch Ausnehmungen für den wenigstens einen Hebel des Vorholers vorzusehen, so daß die für das Gelenk notwendige Auslenkung sichergestellt ist. Um individuellen Bedürfnissen Rechnung tragen zu können, wird zweckmäßigerweise der Abstand der Achse der Anlenkung vor der oberen Plattform einstellbar sein.

An der unteren Plattform erfolgt die Anbindung des Vorholers bevorzugt durch die Führung eines axial federnd gelagerten Kolbens in einer zentralen Aufnahme, welcher Kolben an seinem freien, der zweiten Plattform zugewandten Ende, den wenigstens einen Hebel angelenkt aufweist. Weiter bevorzugt erfolgt die Führung des Kolbens entlang einer Hochachse, die in der Ruheposition im wesentlichen vertikal sich erstreckt.

Es kann weiter für wenigstens eine Achse eine Bremse vorgesehen sein für das Abbremsen einer Drehbewegung um diese Achse. Bei dem Gelenk nach der Erfindung ist das Abbremsen einer einzigen Achse regelmäßig ausreichend, da die Achsen miteinander nach Art einer Gelenkkette miteinander verbunden sind.

Es wird eine Bremse bevorzugt, für die eigenständiger Schutz auch begehrt wird, bei der die Bremskraft durch eine auf dem Gelenk lastende Gewichtskraft hervorgerufen wird. Insbesondere in der Ruheposition ist das Gelenk dann gleichsam verriegelt und sorgt für einen sehr sicheren, komfortablen Stand, da in dieser stationären Ruheposition die Belastung durch das Körpergewicht am größten sein wird.

In konstruktiver Ausgestaltung ist vorgesehen, daß die Achse in einer Fassung geführt ist, aufweisend einen Längsschlitz. Nach Art bekannter Bandbremsen erfolgt daß Abbremsen durch Schließen des Längsschlitzes und damit reibschlüssiger Fassung der Achse. Es hat diese Ausführungsform einer Bremse insbesondere für das Gelenk nach der Erfindung den Vorteil eines äußerst geringen Raumbedarfes.

Es hat sich bewährt, die Gewichtskraft über einen Hebelarm auf die Fassung der Achse einwirken zu lassen, insbesondere über einen Hebelarm, dessen wirksame Hebelarmlänge einstellbar ist, daß heißt insbesondere, das der Ort der Krafteinleitung in den Hebelarm einstellbar ist. Gegebenenfalls kann auch daran gedacht sein, tatsächlich die physikalische Länge des Hebelarmes variabel zu gestalten, beispielsweise durch ein Verdrehen desselben in einem Gewinde. Weiter wird bevorzugt vorgesehen, daß die Gewichtskraft gegen die Kraft einer Feder wirkt. Infolge dieser Maßnahmen kann das Ansprechen der Bremse und der Bremsvorgang selbst optimal den Bedürfnissen des Prothesenträgers angepaßt werden.

Insbesondere ist weiter daran gedacht, daß in einer Position, insbesondere in der Ruheposition, eine Bremskraft voreinstellbar ist, womit den individuellen Bedürfnissen eines Prothesenträgers Rechnung getragen werden kann, indem die nötige Kraft für ein Auslenken insbesondere aus der Ruheposition vorgegeben werden kann.

Das Gelenk nach der Erfindung wird zunächst anhand eines in der Zeichnung dargestellten Funktionsmodelles näher erläutert, welches Funktionsmodell keine Rücksicht auf die tatsächliche Ausführungsform als Kniegelenk in der Prothetik zuläßt. Nachfolgend wird weiter ein bevorzugtes Ausführungsbeispiel eines Gelenkes mit Vorholer und Bremse, in der Ruheposition dargestellt, näher erläutert. In der Zeichnung zeigt:
- Fig. 1: das Funktionsmodell in einer Ruhelage,
- Fig. 2: das Funktionsmodell in einer Auslenkung der oberen Plattform gegenüber der unteren Plattform von 60°,
- Fig. 3: eine maximale Auslenkung des Funktionsmodells von 140°,
- Fig. 4: ein Diagramm zur Erläuterung der Bewegungsabläufe,
- Fig. 5: ein bevorzugtes Ausführungsbeispiel eines Gelenkes nach der Erfindung in einer Fig. 1 entsprechenden Positionierung und Ansicht,
- Fig. 6: eine Ansicht des Gelenkes gem. des Pfeils VI in Fig. 5,
- Fig. 7: einen Schnitt durch das Gelenk gem. der Linie VII, VII in Fig. 6,
- Fig. 8: in einer vergrößerten Darstellung einen Schnitt entsprechend Fig. 7 durch die obere Plattform,
- Fig. 9: eine isometrische Darstellung der unteren Plattform,
- Fig. 10: eine Ansicht der unteren Plattform gem. des Pfeils VI in Fig. 5,
- Fig. 11: einen Schnitt durch die untere Plattform gem. der Linie XI, XI in Fig. 10,
- Fig. 12: einen Schnitt durch die untere Plattform gem. der Linie XII, XII in Fig. 10,
- Fig. 13: eine Draufsicht auf die untere Plattform gem. des Pfeils XIII in Fig. 10,
- Fig. 14: eine isometrische Darstellung der oberen Plattform,
- Fig. 15: eine Ansicht der oberen Plattform gem. des Pfeils VI in Fig. 5,
- Fig. 16: eine Draufsicht auf die obere Plattform gem. des Pfeils XVI in Fig. 15,
- Fig. 17: eine Seitenansicht der oberen Plattform gem. des Pfeils XVII in Fig. 15,
- Fig. 18: einen Schnitt gem. der Linie IIXX, IIXX in Fig. 16,
- Fig. 19: einen Schnitt gem. der Linie IXX, IXX in Fig. 16,
- Fig. 20: eine Fig. 7 entsprechende Seitenansicht eines Anschlußelementes für einen Strumpf,
- Fig. 21: eine Ansicht gem. des Pfeils XXI in Fig. 20,
- Fig. 22: eine Draufsicht auf das Anschlußelement gem. des Pfeils XXII in Fig. 20,
- Fig. 23: eine Unteransicht gem. des Pfeils XXIIV in Fig.24,
- Fig. 24: einen Schnitt durch das Anschlußelement gem. der Linie XXIV, XXIV in Fig. 22,
- Fig. 25: eine Ansicht eines Hebels entsprechend Fig. 6,
- Fig. 26: eine Seitensicht des Hebels,
- Fig. 27: ein Keilstück in einer Seitenansicht entsprechend Fig. 7,
- Fig. 28: eine stirnseitige,
- Fig. 29: eine Draufsicht auf das Keilstück nach Fig. 27,
- Fig. 30: in einer Fig. 7 entsprechenden Ansicht einen Kolbenabschnitt eines Vorholers und
- Fig. 31: eine Draufsicht auf den Kolbenabschnitt gem. des Pfeils XXXI in Fig. 30

Es wird ausdrücklich darauf hingewiesen, daß die nachstehende Beschreibung des Funktionsmodells in ihren Bewegungsabläufen umkehrbar ist. Insofern ist eine Zuordnung von Bezeichnungen weitgehend austauschbar.

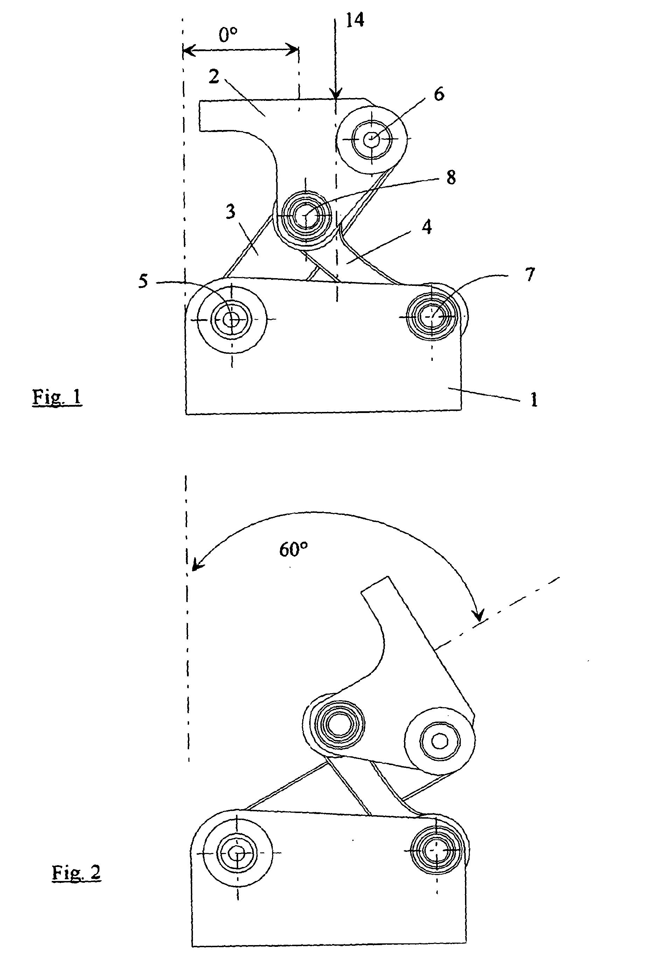

Die Figuren 1 bis 3 zeigen ein Funktionsmodell eines Gelenkes nach der Erfindung in drei verschiedenen Stellungen, wobei die untere Plattform 1 in einer Fixposition gehalten ist.

Entsprechend zeigen die Zeichnungen 2 und 3 eine gegenüber der Plattform 1 angestellte obere Plattform 2.

Die Ausbildungen der Plattformen 1,2 des Funktionsmodells sind hier nicht weiter erläutert, da diese im wesentlichen von der Art des Anschlusses der Prothese bzw. der Aufnahme des Oberschenkels individuell abhängig ausgeführt werden können.

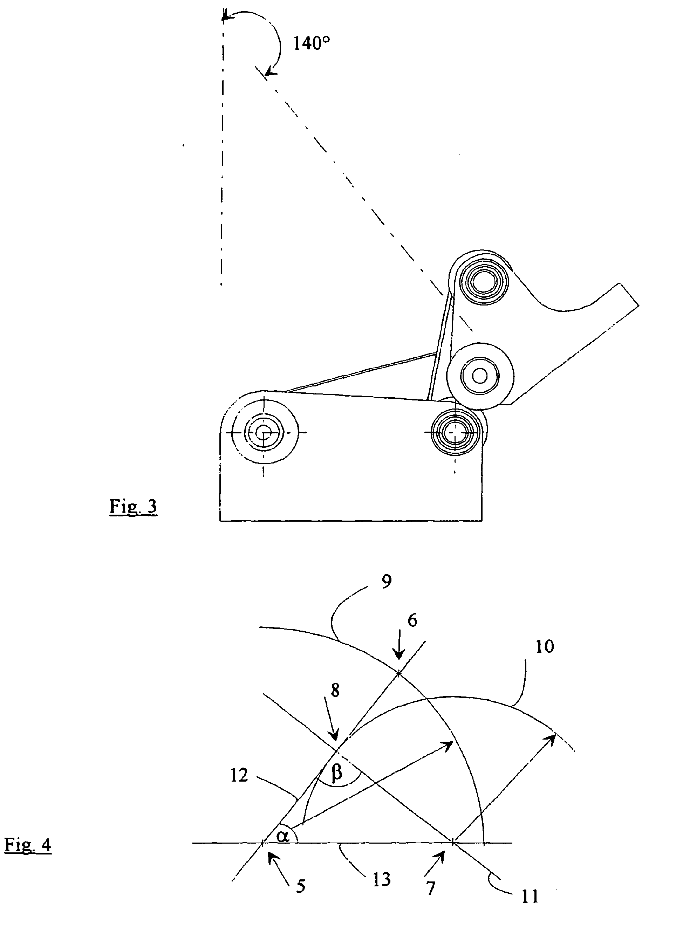

Verbunden sind die Plattformen 1,2 durch wenigsten zwei jeweils endseitig an den Plattformen 1,2 angelenkte Hebel 3,4 unterschiedlicher Länge. Die Achsen 5,6 des Hebels 3 sowie die Achsen 7,8 des zweiten Hebels 4 stehen auf der Zeichenebene senkrecht. Es läßt das Gelenk eine relative Verschwenkung der oberen Plattform 2 gegen die untere Plattform 1 aus der gezeigten Ruheposition gemäß Figur 1 in eine Position maximaler Auslenkung gemäß Figur 3 zu, bei welcher Verschwenkung der Abstand der Plattformen 1,2 sofort mit einer Auslenkung aus der Ruheposition gemäß Figur 1 verkürzt wird. Bei der Betrachtung des zurückgelegten Weges der Achse 6 zeigt sich nämlich, daß diese aufgrund der Anbindung über den Hebel 3 und die Achse 5 an der unteren Plattform 1 sich abwärts auf einer Kreisbahn 9 bewegt, vergleiche auch Figur 4.

Infolge der konstruktiven Ausgestaltung liegt der momentane Drehpunkt bei dem Verschwenken innerhalb des Gelenkes und stellt sich so ein weitestgehend natürlich wirkender Funktionsablauf dar.

Das Schaubild gemäß Figur 4 zeigt weiter, daß in der in Figur 1 gezeigten Ruheposition das Gelenk über diese Stellung hinaus nicht wesentlich weiter verschwenkt werden sollte. Da eine weitere Auslenkung des Hebels 3 in der Figur 4 nach links nicht möglich ist, da bei einem weiteren Absenken der Achse 8 des zweiten Hebels 4 auf der Kreisbahn 10 sich der Abstand zwischen den Achsen 6,8 vergrößern müßte, wird sich die obere Plattform 2 bei einem weiteren Verschwenken in das Gelenk, bei entsprechender konstruktiver Ausgestaltung, eindrehen derart, daß die Oberseite der Plattform 2 zuunterst zu liegen kommt.

Neben Totpunktlagen, selbsthemmenden oder selbstsichernden Gelenkstellungen können insbesondere Anschläge die Ruheposition und/oder die Position der maximalen Auslenkung gemäß Figur 3 festlegen. Ein Einlaufen in diese Positionen erfolgt zweckmäßigerweise gedämpft, so daß es zu keinem harten Auftreffen auf Anschläge bzw. einem abrupten Stillstand kommt. Es kann beispielsweise daran gedacht sein, die Winkelgeschwindigkeit der Achsen abzudämpfen oder geeignete Stoßdämpfer an den Anschlägen auch vorzusehen.

Die Figuren 1 und 4 zeigen weiter, daß in der Ruheposition die die Achsen 5,6 bzw. 7,8 der Hebel 3,4 verbindenden Linien 11,12 annähernd aufeinander senkrecht stehen, d. h., daß der Öffnungswinkel β der Linien 11, 12 etwa 90° beim Ausführungsbeispiel aufweist und damit weniger als 95° beträgt. Weiter liegt die Achse 8 in der Ruheposition auf der Verbindungslinie 12 der Achsen 5,6 des ersten Hebels 3, wobei die Achse 8 etwa mittig zwischen den Achsen 5,6 liegend angeordnet ist.

Beim Ausführungsbeispiel des Funktionsmodells sind die an der unteren Plattform 1 angelenkten ersten Achsen 5,7 im wesentlichen auf gleicher Höhe, einer horizontalen Linie 13 angeordnet. Gegenüber dieser horizontalen Linie 13 beträgt der Öffnungswinkel Alpha der Linie 12 und damit die Anstellung des Hebels 3 gegenüber der horizontalen Linie 13 etwa 52°, einem Winkel Alpha, der in einem Intervall von 45° bis 75° liegt, welches Intervall die Anstellung des vorderen Kreuzbandes gegen einen Unterschenkels eines menschlichen Knies auch beschreibt.

In einer nicht dargestellten Variante gem. Fig. 1 kann jedoch auch eine erste Achse eines Hebels gegenüber der ersten Achse des anderen Hebels tiefer liegend angeordnet sein.

Entsprechend kann der Abstand der ersten Achsen 5,7 der Hebel 3,4 in der unteren Plattform 1 zwischen 36 mm und 62 mm bevorzugt betragen. Auch der Abstand der ersten Achse 5 zur zweiten, an der oberen Plattform 2 angelenkten Achse 6 des ersten Hebels 3 greift die Maße der Anlenkung der Kreuzbänder bzw. deren Längen bei einem menschlichen Knie wieder auf, wenn dieser Abstand zwischen 45 mm und 80 mm beträgt.

Der Abstand der ersten Achse 7 zur zweiten, an der oberen Plattform 2 angelenkten Achse 8 des zweiten Hebels 4 sollte dann zwischen 27 mm und 62 mm betragen, während die zweiten Achsen 6,8 in der Plattform 2 einen Abstand zwischen 16 mm und 62 mm aufweisen sollte. Mit derartigen Abmessungen kann erreicht werden, daß die maximale Auslenkung gemäß Figur 3 etwa 140° beträgt und damit in einem Intervall von 130° bis 150° liegt, das bevorzugt jedoch bis 175° reicht, was der möglichen Schwenkbewegung eines menschlichen Knies weitgehend entspricht.

Infolge dieser Maßnahmen werden sich erster und zweiter Hebel 3,4, zumindest die jeweiligen Verbindungslinien 12,11 der ersten und zweiten Achse 5,6 bzw. 7,8, während eines Verschwenkens ständig kreuzen und liegt so der momentane Drehpunkt ständig innerhalb des Gelenks.

Weiter wird in der Ruhelage gem. Fig. 1 sich die Hauptgewichtsbelastung gemäß Pfeil 14 mit den Hebeln 3,4 kreuzen derart, daß die Linie der Belastung bevorzugt zwischen den zweiten Achsen 6,8 liegt, ggfls. geringfügig in Fig.1 links vor der Achse 8. Infolgedessen wird ein sehr sicherer Stand auch erreicht, da erst mit einer Auslenkung von mehr als etwa 10° die Belastung gem. Pfeil 14 die Verbindungsstrecke zwischen den zweiten Achsen 6,8 nicht mehr schneidet und damit keine hemmende Wirkung des Gelenks eintritt.

Es wird ausdrücklich darauf hingewiesen, daß die Anordnung der Hebel 3,4, beispielsweise auch als Doppelhebel, lediglich beispielhaft ist. So können beispielsweise außerhalb der Plattform 1 liegend zwei erste Hebel 3 endseitig der Achse 5 angeordnet sein, die auch die Plattform 2 einfassen. Alternativ oder zusätzlich ist es gleichfalls möglich, den zweiten Hebel 4 als Doppelhebel, innen oder auch außerhalb der Plattformen 1,2 liegend, auszubilden.

Weiter ist es für die Funktion des Gelenkes nach der Erfindung ohne Bedeutung, ob zwei zweite Hebel einen oder zwei erste Hebel zwischen sich einfassen oder umgekehrt. Auch können Anordnungen gegebenenfalls vorgesehen sein, bei denen, senkrecht zur Zeichenebene, wechselnd erste und zweite Hebel angeordnet sind.

Durch das erfindungsgemäße Gelenk ist eine Gelenkkette gegeben, bei welcher in einer Plattform 1 über eine Achse 5 ein erster Hebel 3 angelenkt ist, der über eine Achse 6 an einer zweiten, oberen Plattform 2 angebunden ist. Innerhalb der Plattform 2 liegt ein weiterer Hebelarm, relativ unbeweglich zu dieser Plattform, als Verbindung hin zu einer weiteren Achse 8, an der ein zweiter Hebel 4 angelenkt ist. Andernends ist der zweite Hebel 4 wieder mit einer Achse 7 an der unteren Plattform 1 festgelegt. Innerhalb der Plattform 1 verläuft ein weiterer Hebelarm, der die ersten Achsen 5,7 miteinander verbindet. Die besondere Kinematik dieses Gelenkes beruht im wesentlichen auch auf den Abmessungen der einzelnen Hebelarme.

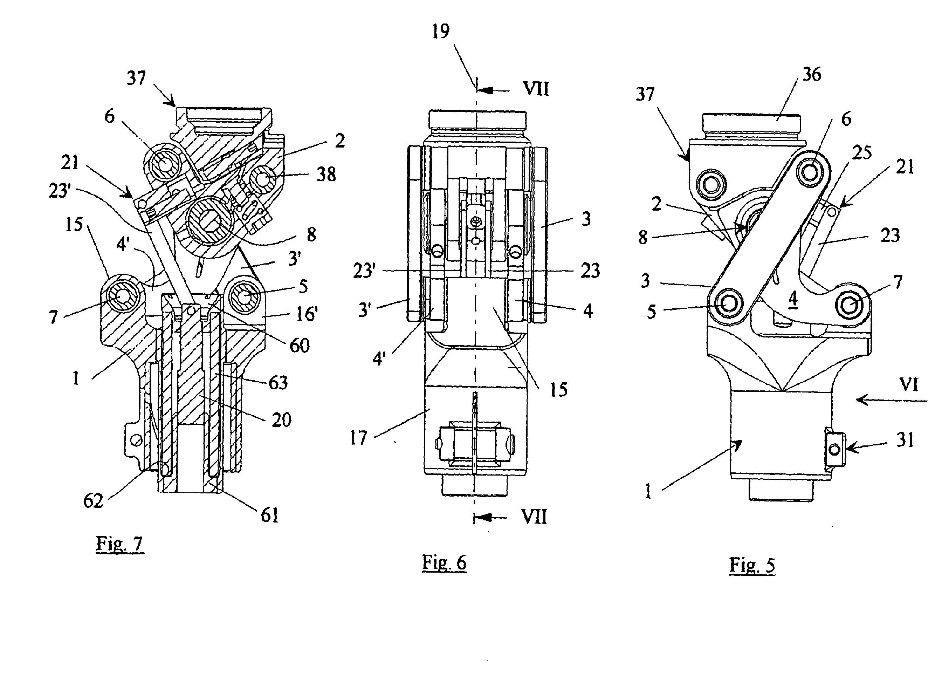

Anhand der nachfolgenden Figuren 5 bis 31 wird ein bevorzugtes Ausführungsbeispiel eines erfindungsgemäßen Gelenks mit Bremse und Vorholer weiter erläutert. Aus Gründen der leichteren Übersicht werden dabei die bisherigen Positionsziffern soweit als möglich beibehalten.

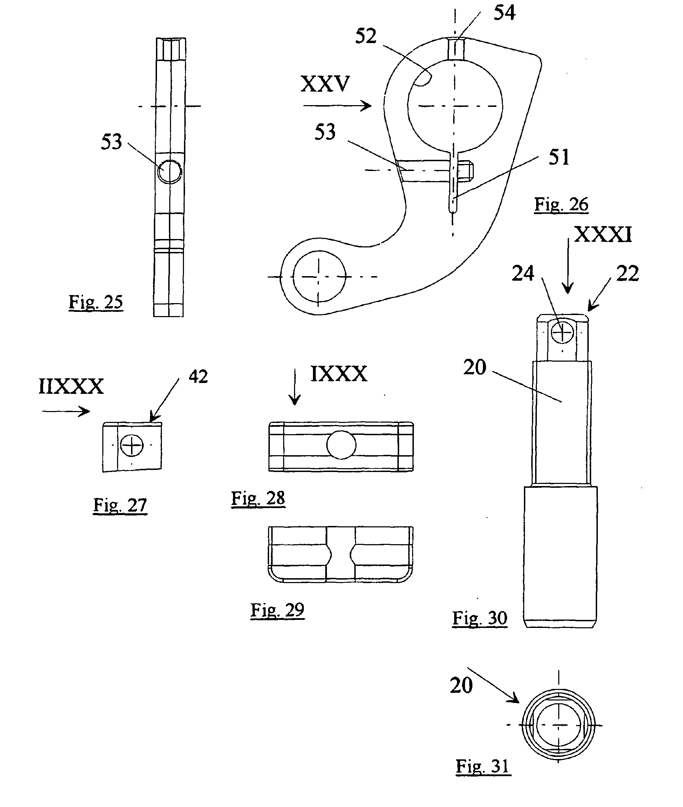

Das in den Fig. 5 bis 7 in seiner Ruheposition gezeigte Gelenk ist im wesentlichen symmetrisch zu seiner Schnittebene gem. Fig. 7 ausgebildet. Es weist eine untere Plattform 1 auf, an der auf einer Achse 5 ein Doppelhebel 3, 3' und einer Achse 7 ein Doppelhebel 4, 4' jeweils angelenkt ist. Die Doppelhebel 4, 4' sind jeweils als Winkelhebel ausgebildet, vgl. auch die Figuren 25, 26. Andernends sind die Hebel, wie erläutert, an einer oberen Plattform 2 an Achsen 6, 8 angelenkt.

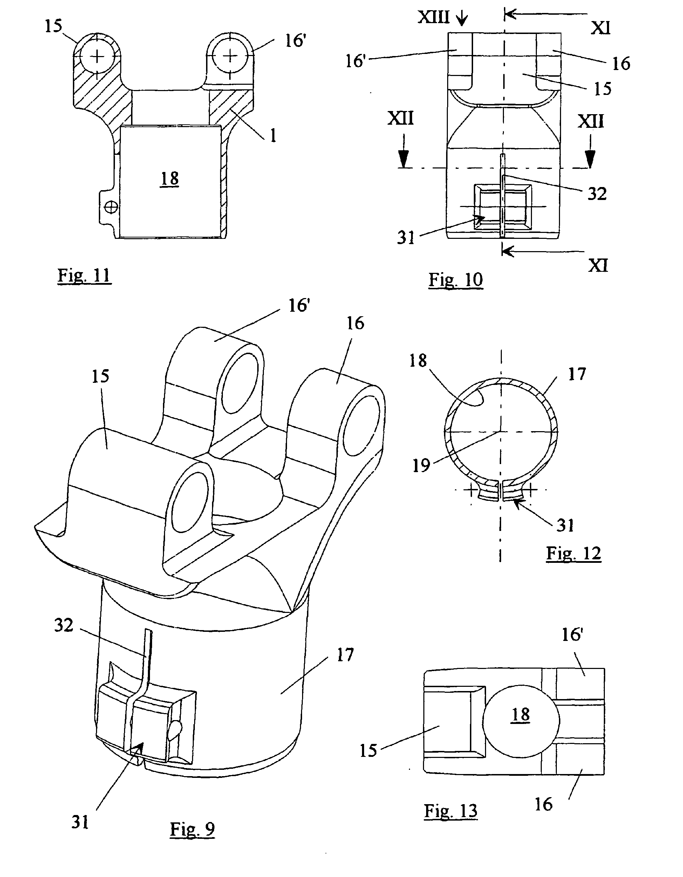

Bei dem Ausführungsbeispiel sind die Achsen 5 bis 8 körperlich ausgebildet und fest mit den Hebeln 3, 3' bzw. 4, 4' verbunden. Die Achsen 5 bis 8 sind in Fassungen 15, 16, 16' der unteren Plattform 2, vgl. Fig. 9, drehbar gelagert. Infolge kann eine Bremse der eingangs erläuterten Art für eine Achse, hier die Achse 8, vorgesehen werden.

Die untere Plattform 1, vgl. Fig. 9 - 13, weist einen holzylindrischen Schaft 17 auf, durch den eine im Querschnitt kreisförmige, zentrale Aufnahme 18 definiert wird, die rotationssymmetrisch zu einer in der Ruhestellung im wesentlichen vertikalen Hochachse 19 ausgebildet ist. In der Aufnahme 18 ist ein Kolbenabschnitt 20, vgl. auch Fig. 30, 31, und daran angeschlossen eine Kronmutter 60 eines Vorholers 21 axial gefedert und verschieblich gelagert, vgl. Fig. 7.

Hierzu ist eine Hülse 61 in die Aufnahme 18 eingebracht, in der der Kolbenabschnitt 20 einerends geführt ist. Andernends durchsetzt der Kolbenabschnitt 20 die Kronmutter 60, die selbst unmittelbar in der Aufnahme 18 geführt sein kann. Zwischen der Kronmutter 60 und einer Ringschulter 62 der Hülse 61 ist eine Feder 63 angeordnet, deren Vorspannung in der gezeigten Ruheposition des Gelenkes durch Verstellen der Kronmutter 60 an dem Kolbenabschnitt 20 einstellbar ist.

An dem freien Ende 22 des Kolbenabschnitts 20, das der oberen Plattform 2 gegenüberliegende, ist ein Doppelhebel 23, 23' angelenkt, wobei die Anlenkachse 24 parallel zu den übrigen Achsen 5 bis 8 ausgebildet ist.

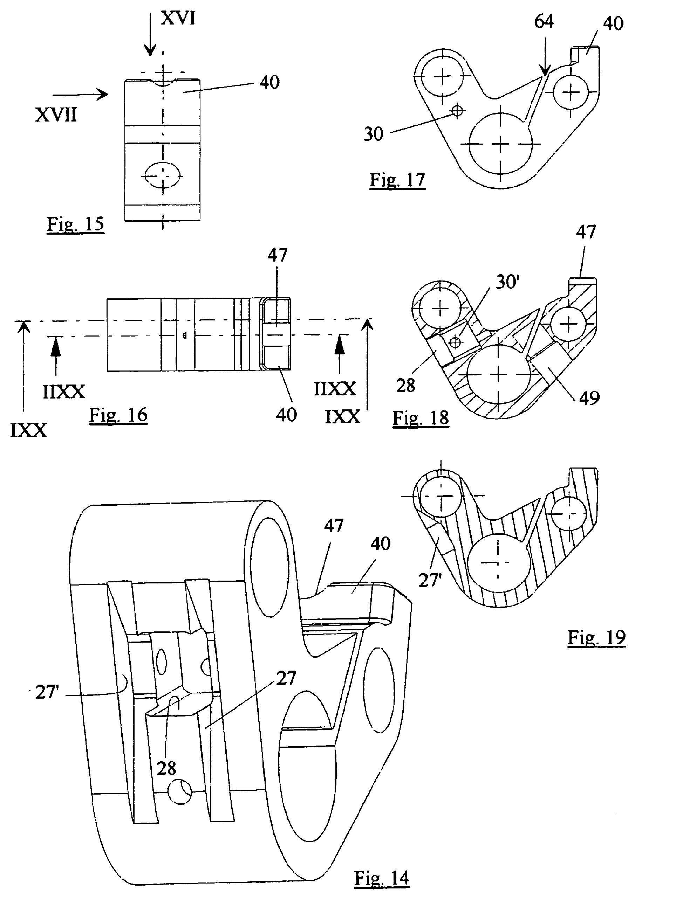

Andernends ist der Doppelhebel 23, 23' an einem der oberen Plattform 2 vorstehenden Ansatz 25 um eine Anlenkachse 26, wieder parallel zu den Achsen 5 bis 8, verschwenkbar angelenkt. Durch das Vorstehen der Anlenkachse 26 vor der oberen Plattform 2 wird ein ungehindertes Verschwenken des Gelenkes bereits weitgehend gewährleistet. Je nah Auslenkung des Gelenkes hat es sich jedoch als zweckmäßig erwiesen, in der oberen Plattform 2 Ausnehmungen 27, 27' für die Hebel 23, 23' noch vorzusehen, vgl. Fig. 14.

Der Ansatz 25 ist gesondert etwa quaderförmig ausgebildet und in einer Ausnehmung 28 in der oberen Plattform 2 aufgenommen. Die Einbringtiefe des Ansatzes 25 in der Ausnehmung 25 und damit der Abstand der Anlenkachse 26 vor der Plattform 2 kann eingestellt werde, bspw. mittels einer, gegebenenfalls auch der Befestigung dienenden Schraube 29. Zusätzlich oder alternativ kann eine Verschraubung des Ansatzes 25 mit der oberen Plattform 2 auch durch Bohrungen 30, 30' erfolgen.

Wird das Gelenk in der gezeigten Ruheposition entlastet, wird die in der Feder 62 gespeicherte Kraft den Kolbenabschnitt 20 des Vorholers 21 nach oben bewegen. Mittels des an dem Kolbenabschnitt 20 angelenkten Doppelhebels 23, 23' wird die Kraft über den Ansatz 25 auf die obere Plattform 2 übertragen und diese gegenüber der unteren Plattform 1 verschwenkt werden. In der Ruheposition wird die Gewichtskraft regelmäßig ausreichend sein, die Feder 62 zu spannen und der Kraft der Feder 62 entgegenzuwirken.

Auch bei dem Vorholer 21 ist grundsätzlich eine Bewegungsumkehr durch eine andere Wahl der federnden Lagerung möglich.

Der hohlzylinderische Schaft 17 dient weiter der Aufnahme eines Anschlußstückes einer nicht dargestellten Prothese. Die Verriegelung von Gelenk und Prothese erfolgt beispielsweise mittels eines einen axialen Spalt 32 überdeckenden Spannschlosses 31.

Bei dem bevorzugten Ausführungsbeispiel ist weiter eine Drehbewegung der Achse 8 unter einer Gewichtskraft abbremsende Bremsvorrichtung der eingangs erläuterten Art vorgesehen.

Dazu ist die Achse 8 in einer Fassung 33 in der oberen Plattform 2 gelagert, die einen Schlitz 34 aufweist, der sich durch die obere Plattform 2 hindurchzieht. Beim Ausführungsbeispiel beschränkt sich daher die Fassung 33 auf ein einfaches axial geschlitztes Röhrchen. Durch ein Verkleinern des Spaltabstandes wird die Achse 8 in der Fassung reibungsgebremst.

Durch die Fortführung des Schlitzes 34 als Spalt 64 in der oberen Plattform 2 wird ein Hebelarm 35 ausgebildet, über den die Gewichtskraft auf die Fassung 33 einwirkt. Aufgebracht wird die Gewichtskraft über einen an sich bekannten Anschluß 36 bspw. für einen Strumpf des Prothesenträgers.

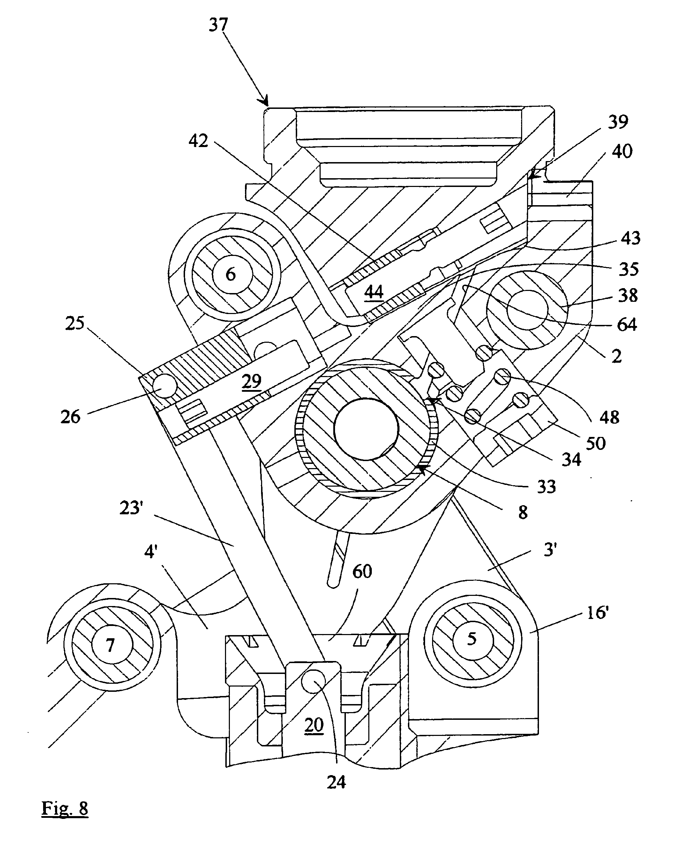

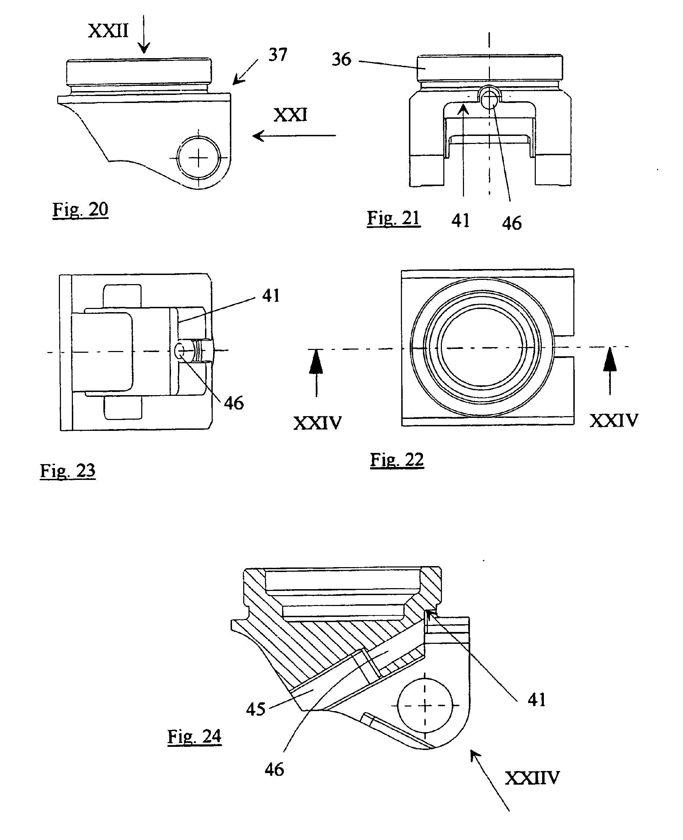

Der Anschluß 36 ist Teil eines gesondert ausgebildeten Anschlußelementes 37, vgl. Fig. 20 bis 24. Es ist dieses Anschlußelement scheinbar um einen Bolzen 38 gegenüber der oberen Plattform 2 verschwenkbar. Tatsächlich ist aber durch die spezielle Art der Anlage, vgl. Pfeil 39 in Fig. 8, zwischen einem Anlagewinkel 40 der oberen Plattform 2 und einem Rückenwinkel 41 des Anschlußelementes 37 ein Verschwenken gegeneinander unmöglich gemacht. Da im übrigen das Anschlußelement 37 frei über der Oberseite des Hebelarms 35 der oberen Plattform 2 steht, wirkt das Anschlußelement 37 aufgrund von Biegespannungen gleichfalls wie ein Hebelarm, der über ein Keilstück 42 die am Anschluß 36 anstehende Gewichtskraft auf den Hebelarm 35 der oberen Plattform 2 überträgt. Hierbei wird die scheinbare Drehachse des Anschlußelementes 37 im wesentlichen durch einen sich in einer entsprechenden Ausnehmung der Plattform 2 abstützenden Fuß 43 markiert.

Das die Kraft weiter übertragende Keilstück 42, vgl. Fig. 27-29, ist veränderbar entlang der Achse der hierzu vorgesehenen Schraube 44 in einer das Keilstück 42 führenden Ausnehmung 45 in dem Anschlußelement 37 positionierbar. Um auch bei einem fertigen Gelenk noch Einstellungen vornehmen zu können, weist das Anschlußelement in axialer Verlängerung der Schraube 44 eine Bohrung 46 noch auf, die sich in einer Mulde 47 des Anlagewinkels 40 der oberen Plattform 2 noch fortsetzt.

Durch das Verschieben des Keilstückes 42, ggfls. zweier Keilstücke gegeneinander, kann weiter eine Vorspannung über den Hebelarm 35 auf die Fassung 33 aufgebracht werden und so eine Bremskraft insbesondere für die Ruheposition voreingestellt werden.

Durch die Veränderung der Position und damit auch der unterschiedlichen Winkeleinstellung der Oberseite des Hebelarms 35 und des Anschlußelementes, bzw. durch unterschiedlich aufbringbare Vorspannungen, wie auch der hierdurch einstellbaren wirksamen Länge des Hebelarms 35, kann eine individuelle Anpassung des Ansprechens und der Charakteristik der Bremse erreicht werden. Dem trägt eine den Hebelarm 35 entlastende und hinsichtlich ihrer Vorspannung einstellbaren Feder 48 bei, die in einer Bohrung 49 von einer Verstellschraube 50 gehalten ist.

Es kann erwünscht sein, daß bei einer großen aufgebrachten Bremskraft es dennoch zu einer Drehbewegung des Gelenkes kommen soll. Hierzu können zwischen der durch die Bremse festgelegten Achse 8 und den Anbindungen an die Hebel 4, 4', alternativ zwischen der oberen Plattform 2 und der Fassung 33 geeignete Maßnahmen bspw. in Form einer Rutschkupplung noch vorgesehen werden. Auch kann es innerhalb der Fassung bei geeigneter Kraftaufbringung und geeigneter Materialwahl zu einem Durchrutschen kommen.

Hierzu trägt die Maßnahme bei, die Anbindung der Hebel 4, 4' an die Achse 8 entsprechend den Fig. 25,26 mittels einer einen Schlitz 51 aufweisenden Aufnahme 52 vorzunehmen. Über eine in einer Bohrungen 53 eingebrachte Schraube, Bolzen oder dergleichen läßt sich dann, unter Verstellen der Schlitzweite, in charakteristischer Weise die von der Anbindung der Hebel 4, 4' an die Achse 8 übertragenen Kräfte einstellen. Eine weitere Bohrung 54 dient der Aufnahme einer Befestigungsschraube für die Fassung 33.

## Patentansprüche

1. Künstliches Kniegelenk, mit einer oberen Plattform (2) und einer unteren Plattform (1), die durch zwei erste und zwei zweite, kürzere Hebel (3,4) miteinander verbunden sind, deren parallele Drehachsen (5,6; 7,8) senkrecht auf einer Ebene stehen, so dass die obere Plattform (2) aus einer Ruheposition in eine Position maximaler Auslenkung relativ verschwenkbar ist, bei welchem Verschwenken der Abstand der Plattformen (1,2) sofort mit einer Auslenkung aus der Ruheposition verkürzt wird und sich die Verbindungslinien (11,12) der Drehachsen (5,6; 7,8) ständig überkreuzen.

2. Kniegelenk nach Anspruch 1, **dadurch gekennzeichnet, dass** während des Verschwenkens der Plattformen gegeneinander der momentane Drehpunkt ständig innerhalb des eigentlichen Kniegelenkes liegt.

3. Kniegelenk nach einem oder mehreren der vorangehenden Ansprüche, **dadurch gekennzeichnet, dass** das Kniegelenk in der Ruheposition und/oder der Position der maximalen Auslenkung selbstsichernd oder selbsthemmend ausgelegt ist oder eine Position durch eine Totpunktstellung des Kniegelenks festgelegt ist.

4. Kniegelenk nach einem oder mehreren der vorangehenden Ansprüche, **dadurch gekennzeichnet, dass** die Ruheposition und/oder die Position der maximalen Auslenkung durch einen oder mehrere Anschläge festgelegt ist bzw. sind.

5. Kniegelenk nach einem oder mehreren der vorangehenden Ansprüche, **gekennzeichnet durch** ein gedämpftes Einlaufen in die Ruheposition und/oder die Position der maximalen Auslenkung.

6. Kniegelenk nach einem oder mehreren der vorangehenden Ansprüche, **dadurch gekennzeichnet, dass** in einer Position, insbesondere in der Ruheposition, der Öffnungswinkel (β) der die Achsen (5,6;7,8) der Hebel (3,4) verbindenden Linien (11,12) weniger als 95° beträgt.

7. Kniegelenk nach einem oder mehreren der vorangehenden Ansprüche, **dadurch gekennzeichnet, dass** in einer Position, insbesondere in der Ruheposition, auf der Verbindungslinie (12) der Achsen (5,6) eines ersten Hebels (3) eine Achse (8) des zweiten Hebels (4) im wesentlichen liegend angeordnet ist.

8. Kniegelenk nach einem oder mehreren der vorangehenden Ansprüche, **dadurch gekennzeichnet, dass** in einer Position, insbesondere in der Ruheposition, die an der unteren Plattform (1) angelenkten ersten Achsen (5,7) der Hebel (3,4) im wesentlichen auf gleicher Höhe (13) angeordnet sind.

9. Kniegelenk nach einem oder mehreren der vorangehenden Ansprüche 1 bis 8, **dadurch gekennzeichnet, dass** in einer Position, insbesondere in der Ruheposition, eine an der unteren Plattform angelenkte erste Achse eines Hebels höher liegend angeordnet ist als die erste Achse des anderen Hebels.

10. Kniegelenk nach einem oder mehreren der vorangehenden Ansprüche, **dadurch gekennzeichnet, dass** in einer Position, insbesondere in der Ruheposition, der erste Hebel (3) gegenüber der die ersten Achsen (5,7) verbindenen Linie (13) einen Winkel (α) zwischen 45° und 75°, insbesondere zwischen 50° und 60° aufspannt.

11. Kniegelenk nach einem oder mehreren der vorangehenden Ansprüche, **dadurch gekennzeichnet, dass** der Abstand der ersten Achsen (5,7) zwischen 36 mm und 62 mm beträgt.

12. Kniegelenk nach einem oder mehreren der vorangehenden Ansprüche, **dadurch gekennzeichnet, dass** der Abstand der ersten Achse (5) zur zweiten, an der oberen Plattform (2) angelenkten Achse (6) des ersten Hebels (3) zwischen 45 mm und 80 mm beträgt.

13. Kniegelenk nach einem oder mehreren der vorangehenden Ansprüche, **dadurch gekennzeichnet, dass** der Abstand der ersten Achse (7) zur zweiten, an der oberen Plattform (2) angelenkten Achse (8) des zweiten Hebels (4) zwischen 27 mm und 62 mm beträgt.

14. Kniegelenk nach einem oder mehreren der vorangehenden Ansprüche, **dadurch gekennzeichnet, dass** der Abstand der zweiten Achsen (6,8) zwischen 16 mm und 46 mm beträgt.

15. Kniegelenk nach einem oder mehreren der vorangehenden Ansprüche, **dadurch gekennzeichnet, dass** die maximale Auslenkung des Kniegelenkes zwischen 130° und 175° beträgt.

16. Kniegelenk nach einem oder mehreren der vorangehenden Ansprüche, **dadurch gekennzeichnet, dass** zwischen oberer (2) und unterer Plattform (1) ein Vorholer (21) angeordnet ist.

17. Kniegelenk nach einem oder mehreren der vorangehenden Ansprüche, **dadurch gekennzeichnet, dass** in der Ruheposition der Vorholer (21) zwischen den Achsen (6, 8) der oberen Platform (2) angebunden ist.

18. Kniegelenk nach einem oder mehreren der vorangehenden Ansprüche, **dadurch gekennzeichnet, dass** wenigstens ein Hebel (23, 23') des Vorholers (21) an einem der oberen Plattform (2) vorstehenden Ansatz (25) angelenkt ist.

19. Kniegelenk nach einem oder mehreren der vorangehenden Ansprüche, **dadurch gekennzeichnet, dass** der Abstand der Achse (26) der Anlenkung vor der oberen Plattform (2) einstellbar ist.

20. Kniegelenk nach einem oder mehreren der vorangehenden Ansprüche, **dadurch gekennzeichnet, dass** die untere Plattform (1) eine zentrale Aufnahme (18) für einen axial federnd gelagerten Kolben (20) aufweist, an dessen freien Ende (22) der wenigstens eine Hebel (23, 23') angelenkt ist.

21. Kniegelenk nach einem oder mehreren der vorangehenden Ansprüche, **dadurch gekennzeichnet, dass** für wenigstens eine Achse (8) eine Bremse vorgesehen ist für ein Abbremsen einer Drehbewegung um diese Achse (8).

22. Kniegelenk nach Anspruch 21, **dadurch gekennzeichnet, dass** die Bremskraft durch eine auf dem Kniegelenk lastende Gewichtskraft hervorgerufen wird.

23. Kniegelenk nach Anspruch 22, **dadurch gekennzeichnet, dass** die Achse (8) in einer mit einem axialen Schlitz (34) versehenen Fassung (33) geführt ist.

24. Kniegelenk nach einem oder mehreren der vorangehenden Ansprüche 22 und 23, **dadurch gekennzeichnet, dass** die Gewichtskraft über einen Hebelarm (35) auf die Fassung (33) einwirkt.

25. Kniegelenk nach Anspruch 24, **dadurch gekennzeichnet, dass** der wirksame Hebelarm einstellbar ist.

26. Kniegelenk nach einem oder mehreren der vorangehenden Ansprüche 21 bis 25, **dadurch gekennzeichnet, dass** in einer Position, insbesondere in der Ruheposition eine Bremskraft voreinstellbar ist.

27. Kniegelenk nach Anspruch 24, **dadurch gekennzeichnet, dass** die Gewichtskraft gegen die Kraft einer Feder (48) wirkt.

## Claims

1. An artificial knee joint having an upper platform (2) and a lower platform (1) which are connected to one another by two first and two second shorter levers (3, 4), their parallel axles of rotation (5, 6; 7, 8) being perpendicular to a plane, so that the upper platform (2) is pivotable out of a resting position into a position of maximal deflection relatively, in which pivoting the distance between the platforms (1, 2) is shortened immediately with deflection out of the resting position, and the connecting lines (11, 12) of the axles of rotation (5, 6; 7,8) intersect repeatedly.

2. The knee joint according to Claim 1, **characterized in that** the instantaneous fulcrum during pivoting of the platforms with respect to one another is constantly within the actual knee joint.

3. The knee joint according to any one or more of the preceding claims, **characterized in that** the knee joint is designed to be self-locking or interlocking in the resting position and/or in the position of maximal deflection or a position is defined by a dead point position of the knee joint.

4. The knee joint according to any one or more of the preceding claims, **characterized in that** the resting position and/or the position of the maximal deflection is/are defined by one or more stops.

5. The knee joint according to any one or more of the preceding claims, **characterized by** an attenuated approach to the resting position and/or the position of maximal deflection.

6. The knee joint according to any one or more of the preceding claims, **characterized in that** the opening angle (β) of the lines (11, 12) connecting the axles (5, 6; 7, 8) of the levers (3, 4) amounts to less than 95° in one position, in particular in the resting position.

7. The knee joint according to any one or more of the preceding claims, **characterized in that** an axle (8) of the second lever (4) is arranged essentially horizontally on the connecting line (12) of the axles (5, 6) of a first lever (3) in one position, in particular in the resting position.

8. The knee joint according to any one or more of the preceding claims, **characterized in that** the first axles (5, 7) of the levers (3, 4), which are hinge-connected to the lower platform (1), are arranged essentially at the same height (13) in one position, in particular in the resting position.

9. The knee joint according to any one or more of the preceding claims 1 through 8, **characterized in that** a first axle of a lever that is hinge-connected to the lower platform is arranged at a higher level than the first axle of the other lever in one position, in particular in the resting position.

10. The knee joint according to any one or more of the preceding claims, **characterized in that** the first lever (3) spans an angle (α) between 45° and 75°, in particular between 50° and 60°, with respect to the line (13) connecting the first axles (5, 7) in one position, in particular in the resting position.

11. The knee joint according to any one or more of the preceding claims, **characterized in that** the distance between the first axles (5, 7) is between 36 mm and 62 mm.

12. The knee joint according to any one or more of the preceding claims, **characterized in that** the distance of the first axle (5) from the second axle (6) of the first lever (3) which is hinge-connected to the upper platform (2) amounts to between 45 mm and 80 mm.

13. The knee joint according to any one or more of the preceding claims, **characterized in that** the distance of the first axle (7) from the second axle (8) of the second lever (4) which is hinge-connected to the upper platform (2) amounts to between 27 mm and 62 mm.

14. The knee joint according to any one or more of the preceding claims, **characterized in that** the distance of the second axles (6, 8) is between 16 mm and 46 mm.

15. The knee joint according to any one or more of the preceding claims, **characterized in that** the maximal deflection of the knee joint is between 130° and 175°.

16. The knee joint according to any one or more of the preceding claims, **characterized in that** a advancing device (21) is arranged between the upper platform (2) and the lower platform (1).

17. The knee joint according to any one or more of the preceding claims, **characterized in that** in the resting position the advancing device (21) is connected between the axles (6, 8) of the upper platform (2).

18. The knee joint according to any one or more of the preceding claims, **characterized in that** at least one lever (23, 23') of the advancing device (21) is hinge-connected to a shoulder (25) protruding beyond the upper platform (2).

19. The knee joint according to any one or more of the preceding claims, **characterized in that** the distance from the axle (26) of the articulated connection to the upper platform (2) is adjustable.

20. The knee joint according to any one or more of the preceding claims, **characterized in that** the lower platform (1) has a central receptacle (18) for an axially spring-mounted piston (20), at least one lever (23, 23') being connected to its free end (22) with an articulated connection.

21. The knee joint according to any one or more of the preceding claims, **characterized in that** a brake is provided for at least one axle (8) for braking a rotational movement about this axle (8).

22. The knee joint according to Claim 21, **characterized in that** the braking force is induced by a loaded weight acting on the knee joint.

23. The knee joint according to Claim 22, **characterized in that** the axle (8) is guided in a frame (33) which is provided with an axial slot (34).

24. The knee joint according to any one or more of the preceding Claims 22 and 23, **characterized in that** the weight acts on the frame (33) via a lever arm (35).

25. The knee joint according to Claim 24, **characterized in that** the active lever arm is adjustable.

26. The knee joint according to any one or more of the preceding Claims 21 through 25, **characterized in that** a braking force is preadjustable in one position, in particular in the resting position.

27. The knee joint according to Claim 24, **characterized in that** the weight acts against the force of a spring (48).

## Revendications

1. Articulation à genouillère artificielle, avec une plate-forme supérieure (2) et une plate-forme inférieure (1) reliées entre elles au moyen de deux premiers leviers et de deux deuxièmes leviers plus courts (3, 4), dont les axes de rotation parallèles (5,6 ; 7,8) se trouvent à la verticale sur une étendue, de façon à ce que la plate-forme supérieure (2) puisse être pivotée de manière relative d'une position de repos vers une position de déflexion maximale, un tel pivotement entraînant immédiatement un raccourcissement de l'écart entre les plate-formes (1, 2) par une déflexion hors de la position de repos, ainsi qu'un croisement constant des lignes de jonction (11, 12) des axes de rotation (5,6 ; 7,8).

2. Articulation de genouillère selon la revendication 1, **caractérisée en ce que** pendant le pivotement des plate-formes l'une contre l'autre, le point de rotation momentané se trouve en permanence à l'intérieur de l'articulation de genouillère effective.

3. Articulation de genouillère selon l'une ou plusieurs des précédentes revendications, **caractérisée en ce que** dans la position de repos et/ou dans la position de déflexion maximale, l'articulation de genouillère est conçue de façon auto-sécurisée ou auto-bloquante, ou en ce qu'une position de point mort est déterminée pour l'articulation de genouillère.

4. Articulation de genouillère selon une ou plusieurs des précédentes revendications, **caractérisée en ce que** la position de repos et/ou la position de déflexion maximale sont déterminées par une ou plusieurs butées.

5. Articulation de genouillère selon l'une ou plusieurs des précédentes revendications, **caractérisée par** une mise en place modérée en position de repos et/ou en position de déflexion maximale.

6. Articulation de genouillère selon l'une ou plusieurs des précédentes revendications, **caractérisée en ce que** dans une position, notamment dans la position de repos, l'angle d'ouverture (β) des lignes de jonction (11, 12) reliant les axes (5,6 ; 7,8) des leviers (3, 4) mesure moins de 95°.

7. Articulation de genouillère selon l'une ou plusieurs des précédentes revendications, **caractérisée en ce que** dans une position, notamment dans la position de repos, un axe (8) du deuxième levier (4) est disposé essentiellement de façon couchée sur la ligne de jonction (12) des axes (5, 6) d'un premier levier (3).

8. Articulation de genouillère selon l'une ou plusieurs des précédentes revendications, **caractérisée en ce que** dans une position, notamment dans la position de repos, les premiers axes (5, 7) des leviers (3, 4) articulés sur la plate-forme inférieure (1) sont disposés essentiellement à la même hauteur (13).

9. Articulation de genouillère selon l'une ou plusieurs des précédentes revendications 1 à 8, **caractérisée en ce que** dans une position, notamment dans la position de repos, un premier axe de levier articulé à la plate-forme inférieure est disposée plus haut que le premier axe de l'autre levier.

10. Articulation de genouillère selon l'une ou plusieurs des précédentes revendications, **caractérisée en ce que** dans une position, notamment dans la position de repos, le premier levier (3) forme un angle (α) mesurant entre 45° et 75°, notamment entre 50° et 60°, par rapport à la ligne de jonction (13) reliant les premiers axes (5, 7).

11. Articulation de genouillère selon l'une ou plusieurs des précédentes revendications, **caractérisée en ce que** l'écart entre les premiers axes (5, 7) mesure entre 36 mm et 62 mm.

12. Articulation de genouillère selon l'une ou plusieurs des précédentes revendications, **caractérisée en ce que** l'écart du premier axe (5) par rapport au deuxième axe (6) du premier levier (3), qui est articulé sur la plate-forme supérieure (2), mesure entre 45 mm et 80 mm.

13. Articulation de genouillère selon l'une ou plusieurs des précédentes revendications, **caractérisée en ce que** l'écart du premier axe (7) par rapport au deuxième axe (8) du deuxième levier (8), qui est articulé sur la plate-forme supérieure (2), mesure entre 27 mm et 62 mm.

14. Articulation de genouillère selon l'une ou plusieurs des précédentes revendications, **caractérisée en ce que** l'écart entre les deuxièmes axes (6, 8) mesure entre 16 mm et 46 mm.

15. Articulation de genouillère selon l'une ou plusieurs des précédentes revendications, **caractérisée en ce que** la déflexion maximale de l'articulation de genouillère mesure entre 130° et 175°.

16. Articulation de genouillère selon l'une des précédentes revendications, **caractérisée en ce qu'**un récupérateur (21) est prévu entre la plate-forme supérieure (2) et la plate-forme inférieure (1).

17. Articulation de genouillère selon l'une ou plusieurs des précédentes revendications, **caractérisée en ce que** dans la position de repos, le récupérateur (21) est attaché entre les axes (6, 8) de la plate-forme supérieure (2).

18. Articulation de genouillère selon l'une ou plusieurs des précédentes revendications, **caractérisée en ce qu'**au moins un levier (23, 23') du récupérateur (21) est articulé sur une saillie (25) dépassant de la plate-forme supérieure (2).

19. Articulation de genouillère selon l'une ou plusieurs des précédentes revendications, **caractérisée en ce que** l'écart de l'axe (26) de l'articulation devant la plate-forme supérieure (2) peut être ajusté.

20. Articulation de genouillère selon l'une ou plusieurs des précédentes revendications, **caractérisée en ce que** la plate-forme inférieure (1) comporte une admission centrale (18) pour un piston (20) disposé de façon élastique dans l'axe, sur l'extrémité libre (22) duquel est articulé le levier (23, 23').

21. Articulation de genouillère selon l'une ou plusieurs des précédentes revendications, **caractérisée en ce qu'**un frein est prévu pour un axe (8) au moins, pour freiner une rotation autour de l'axe (8).

22. Articulation de genouillère selon la revendication 21, **caractérisée en ce que** la force de freinage est produite par une force massique pesant sur l'articulation de genouillère.

23. Articulation de genouillère selon la revendication 22, **caractérisée en ce que** l'axe (8) est introduit dans une monture (33) pourvue d'une fente axiale (34).

24. Articulation de genouillère selon l'une ou plusieurs des précédentes revendications 22 et 23, **caractérisée en ce que** la force massique agit sur la monture (33) par le biais d'un bras de levier (35).

25. Articulation de genouillère selon la revendication 24, **caractérisée en ce que** le bras de levier actif est réglable.

26. Articulation de genouillère selon l'une ou plusieurs des précédentes revendications 21 à 25, **caractérisée en ce que** dans une position, notamment dans la position de repos, une force de freinage peut être pré-réglée.

27. Articulation de genouillère selon la revendication 24, **caractérisée en ce que** la force massique agit contre la force d'un ressort (48).
